# EUROPEAN PATENT APPLICATION

(11) **EP 4 174 086 A1**
(43) Date of publication of application: **03.05.2023**
(21) Application number: 21834393.7
(22) Date of filing: 18.06.2021
(51) Int. Cl.: C07K 16/18, G01N 33/574, A61P 35/00, A61K 39/00

(54) **ANTIBODY SPECIFICALLY BINDING TO LGALS3BP, AND USE THEREOF**

(30) Priority: 30.06.2020 KR 20200080105; 14.06.2021 KR 20210076705
(71) Applicant: The Asan Foundation, Seoul 05505 (KR); University Of Ulsan Foundation For Industry Cooperation, Ulsan 44610 (KR)
(72) Inventor: CHANG, Su Hwan, Namyangju-si Gyeonggi-do 12222 (KR); KIM, Song-Cheol, Seoul 05507 (KR); CHOI, Yeon Sook, Seoul 05516 (KR); KIM, Hyo Ri, Seoul 05507 (KR)
(74) Representative: Roos, Peter
(86) International application number: PCT/KR2021/007673
(87) International publication number: WO 2022/005068

(57) **Abstract**

The present invention relates to an antibody specifically binding to galectin 3-binding protein (LGALS3BP), and a use thereof, and, more specifically, to an antibody specifically binding to LGALS3BP, comprising heavy chain CDRs and light chain CDRs of specific sequences, or to an antigen-binding fragment thereof. It has been verified that the antibody specifically binding to LGALS3BP, according to the present invention, has the effect of inhibiting cancer growth and metastasis in an animal model with cancer, and thus is expected to be effectively useful for alleviating or treating cancer.

## Description

### Technical Field

The present disclosure relates to an antibody specifically binding to galectin 3-binding protein (LGALS3BP) and a use thereof.

### Background Art

Despite surgical removal, radiation therapy, chemotherapy, and immunotherapy to treat cancer as well as many studies, it is reported that more than 50% of all cancer patients die without being cured. The reason for this is that the cancer recurs due to the failure to remove the microscopically metastasized cancer cells even after surgical resection, or despite the development of various anti-cancer agents, in the cancer treatment using anti-cancer drugs, the death of cancer cells is not induced by anti-cancer agents or cancer cells that become resistant to the anti-cancer agents rapidly proliferate during or after treatment, although the tumor appears to respond and shrink at an early stage. In addition, radiation therapy also causes resistance of cancer cells to radiation, and thus complete recovery is hardly expected. Anticancer immunotherapy is a method of inducing an immune response using cancer-specific antigens or cancer-related antigens to eliminate antigen-bearing cancer cells using immune cells, but although it is regarded as an innovative cancer treatment method due to little side effects, the clinical effectiveness is not high.

Antibody drugs have been continuously researched and developed for clinical applicability since the first monoclonal antibody was derived in 1975, wherein the fundamental core of the potential for drug development is the mechanism of very strongly and selectively controlling specific biological responses using the properties of an antibody that has strong binding affinity against antigen and high binding specificity. In particular, antibody drugs have relatively few side effects and excellent therapeutic efficacy due to high binding specificity and stability in vivo compared to conventional chemical compound-based therapeutic agents, and thus the field of developing therapeutic agents using antibody substances is spotlighted as the next-generation core field of new drug R&D. Accordingly, antibody drugs showing outstanding therapeutic efficacy compared to side effects in the treatment of various blood cancers and solid cancers have been continuously developed to be used in the clinical practice.

### Disclosure of the Invention

### Technical Goals

An object of the present disclosure is to provide an antibody or antigen-binding fragment thereof specifically binding to galectin 3-binding protein (LGALS3BP).

Another object of the present disclosure is to provide a nucleic acid molecule encoding the antibody or antigen-binding fragment thereof, a recombinant expression vector including the nucleic acid molecule, and a cell transformed with the recombinant expression vector.

Another object of the present disclosure is to provide a composition for diagnosing cancer, including the antibody or antigen-binding fragment thereof as an active ingredient.

Another object of the present disclosure is to provide a pharmaceutical composition for preventing or treating cancer, including the antibody or antigen-binding fragment thereof as an active ingredient.

Another object of the present disclosure is to provide a pharmaceutical composition for preventing or treating cancer metastasis, including the antibody or antigen-binding fragment thereof as an active ingredient.

### Technical Solutions

In order to achieve the above object, the present disclosure provides an antibody or antigen-binding fragment thereof specifically binding to galectin 3-binding protein (LGALS3BP), including a heavy chain variable region including a heavy chain CDR1 having an amino acid sequence represented by SEQ ID NO: 1, a heavy chain CDR2 having an amino acid sequence represented by SEQ ID NO: 2, and a heavy chain CDR3 having an amino acid sequence represented by SEQ ID NO: 3; and a light chain variable region including a light chain CDR1 having an amino acid sequence represented by SEQ ID NO: 4, a light chain CDR2 having an amino acid sequence represented by SEQ ID NO: 5, and a light chain CDR3 having an amino acid sequence represented by SEQ ID NO: 6.

In addition, the present disclosure provides an antibody or antigen-binding fragment thereof specifically binding to LGALS3BP, including a heavy chain variable region including a heavy chain CDR1 having an amino acid sequence represented by SEQ ID NO: 9, a heavy chain CDR2 having an amino acid sequence represented by SEQ ID NO: 10, and a heavy chain CDR3 having an amino acid sequence represented by SEQ ID NO: 11; and a light chain variable region including a light chain CDR1 having an amino acid sequence represented by SEQ ID NO: 12, a light chain CDR2 having an amino acid sequence represented by SEQ ID NO: 13, and a light chain CDR3 having an amino acid sequence represented by SEQ ID NO: 14.

The present disclosure also provides a nucleic acid molecule encoding the antibody or antigen-binding fragment thereof.

In addition, the present disclosure provides a recombinant expression vector including the nucleic acid molecule.

In addition, the present disclosure provides a cell transformed with the recombinant expression vector.

In addition, the present disclosure provides a composition for diagnosing cancer, including the antibody or antigen-binding fragment thereof as an active ingredient.

In addition, the present disclosure provides a pharmaceutical composition for preventing or treating cancer, including the antibody or antigen-binding fragment thereof as an active ingredient.

In addition, the present disclosure provides a pharmaceutical composition for preventing or treating cancer metastasis, including the antibody or antigen-binding fragment thereof as an active ingredient.

### Advantageous Effects

The present disclosure relates to an antibody specifically binding to galectin 3-binding protein (LGALS3BP) and a use thereof, and more specifically, an antibody or antigen-binding fragment thereof specifically binding to LGALS3BP which includes a heavy chain CDR and a light chain CDR of a specific sequence. An antibody that specifically binds to LGALS3BP according to the present disclosure is expected to be usefully applied for improvement or treatment of cancer diseases, as the effect of inhibiting cancer growth and metastasis has been verified in an animal model with cancer.

### Brief Description of Drawings

FIG. 1 shows results of discovering LGALS3BP using proteomic technique in cancer tissues of a pancreatic cancer patient-derived xenograft model.
FIG. 2 shows results of identifying inhibition of cancer development and growth when the expression of LGALS3BP is inhibited in pancreatic cancer cells.
FIG. 3 shows results of identifying reduction in cell migration and invasion ability when the expression of LGALS3BP is inhibited in pancreatic cancer cells.
FIG. 4 shows a schematic diagram of screening of LGALS3BP antibody using the phage display technique.
FIG. 5 shows results of identifying that antibodies #67 and #84 have excellent binding ability and reduce the migrating ability of cancer cells through functional analysis of antibody candidates.
FIG. 6 shows results of verifying the effect of LGALS3BP antibody in inhibiting cancer growth and metastasis in an animal model with pancreatic cancer.
FIG. 7 shows a result of ELISA of four types of antibodies against LGalS3BP.
FIGS. 8 and 9 show the metastatic ability inhibitory effect of humanized LGALS3BP antibody.
FIG. 10 is a schematic diagram illustrating the mechanism of inhibiting cancer metastasis through Gal-3BP blocking by LGALS3BP antibody.

### Best Mode for Carrying Out the Invention

The present disclosure provides an antibody or antigen-binding fragment thereof specifically binding to galectin 3-binding protein (LGALS3BP), including a heavy chain variable region including a heavy chain CDR1 having an amino acid sequence represented by SEQ ID NO: 1, a heavy chain CDR2 having an amino acid sequence represented by SEQ ID NO: 2, and a heavy chain CDR3 having an amino acid sequence represented by SEQ ID NO: 3; and a light chain variable region including a light chain CDR1 having an amino acid sequence represented by SEQ ID NO: 4, a light chain CDR2 having an amino acid sequence represented by SEQ ID NO: 5, and a light chain CDR3 having an amino acid sequence represented by SEQ ID NO: 6.

Preferably, the heavy chain variable region may include an amino acid sequence represented by SEQ ID NO: 7, and the light chain variable region may include an amino acid sequence represented by SEQ ID NO: 8, but are not limited thereto.

In addition, the present disclosure provides an antibody or antigen-binding fragment thereof specifically binding to LGALS3BP, including a heavy chain variable region including a heavy chain CDR1 having an amino acid sequence represented by SEQ ID NO: 9, a heavy chain CDR2 having an amino acid sequence represented by SEQ ID NO: 10, and a heavy chain CDR3 having an amino acid sequence represented by SEQ ID NO: 11; and a light chain variable region including a light chain CDR1 having an amino acid sequence represented by SEQ ID NO: 12, a light chain CDR2 having an amino acid sequence represented by SEQ ID NO: 13, and a light chain CDR3 having an amino acid sequence represented by SEQ ID NO: 14.

Preferably, the heavy chain variable region may include an amino acid sequence represented by SEQ ID NO: 15, and the light chain variable region may include an amino acid sequence represented by SEQ ID NO: 16, but are not limited thereto.

Preferably, the heavy chain variable region may include an amino acid sequence represented by SEQ ID NO: 21, and the light chain variable region may include an amino acid sequence represented by SEQ ID NO: 22, an amino acid sequence represented by SEQ ID NO: 23, or an amino acid sequence represented by SEQ ID NO: 24, wherein the antibody may be a humanized antibody.

Meanwhile, the amino acid sequences of the antibody CDRs, the heavy chain variable region and the light chain variable region of the present disclosure are listed in Tables 2 and 4, and SEQ ID NOs are indicated for each sequence.

As used herein, the term "antibody" refers to a protein molecule that includes an immunoglobulin molecule having immunological reactivity with a specific antigen and serves as a receptor specifically recognizing an antigen, including, for example, all the monoclonal antibody, polyclonal antibody, full-length antibody, and antibody fragments. Also, the term "antibody" may include bivalent or bispecific molecules (e.g., bispecific antibodies), diabodies, triabodies, or tetrabodies.

As used herein, the term "monoclonal antibody" refers to an antibody molecule of a single molecular composition obtained from a population of substantially identical antibodies, and such a monoclonal antibody is different from a polyclonal antibody capable of binding to multiple epitopes and exhibits single binding properties and affinity for a specific epitope. As used herein, the term "full-length antibody" has a structure having two full-length light chains and two full-length heavy chains, wherein each light chain is connected to the heavy chain by a disulfide bond. The heavy chain constant region has gamma (γ), mu (µ), alpha (α), delta (δ) and epsilon (ε) types and gamma 1 (γ1), gamma 2 (y2), gamma 3 (y3), gamma 4 (y4), alpha 1 (α1), and alpha 2 (α2) as subclasses. The constant region of the light chain has a kappa (κ) and a lambda (λ) type. IgG is a subtype, including IgG1, IgG2, IgG3, and IgG4.

As used herein, the term "humanized antibody" refers to an antibody that is non-immunogenic to humans or has reduced immunogenicity. The humanized antibody is an altered antibody with an amino acid sequence altered, and the amino acid sequence of the antibody may be reconstituted for a desired purpose. These possible alterations are numerous, ranging from alteration of one or a few amino acids to complete reconstitution of the variable and/or constant regions of an antibody. In general, alteration of the variable region is performed to increase antigen binding ability and affinity, whereas that in the constant region is performed to improve complement fixation, interaction with membranes, and intracellular actions such as functions of other effectors.

As used herein, the term "heavy chain" may all include a full-length heavy chain and fragment thereof, including a variable region VH having an amino acid sequence with sufficient variable region sequence to give specificity to an antigen and three constant regions CH1, CH2 and CH3. In addition, as used herein, the term "light chain" may include both a full-length light chain and fragment thereof, including a variable region VL and a constant region CL, including an amino acid sequence having a sufficient variable region sequence to give specificity to an antigen.

The terms "fragment", "antibody fragment" and "antigen-binding fragment" as used herein refer to any fragment of an antibody of the present disclosure that has the antigen-binding function of the antibody and are used interchangeably. Exemplary antigen binding fragments include Fab, Fab', F(ab')2 and Fv, but are not limited thereto.

The antibody or antigen-binding fragment thereof of the present disclosure may include not only the sequence of the antibody described herein, but also a biological equivalent thereof, within the range capable of deriving the ability to specifically bind to LGALS3BP. For example, additional alteration may be given to the amino acid sequence of the antibody to further improve the binding affinity and/or other biological properties of the antibody. Such alterations include, for example, deletions, insertions and/or substitutions of amino acid sequence residues of the antibody. Such amino acid variations are made based on the relative similarity of amino acid side chain substituents, such as hydrophobicity, hydrophilicity, charge, and size. By analysis of the size, shape and type of amino acid side chain substituents, it may be indicated that arginine, lysine and histidine are all positively charged residues; alanine, glycine and serine have similar sizes; and phenylalanine, tryptophan and tyrosine have similar shapes. Therefore, based on this, arginine, lysine and histidine; alanine, glycine and serine; and phenylalanine, tryptophan and tyrosine may be considered to be biologically functional equivalents.

The present disclosure also provides a nucleic acid molecule encoding the antibody or antigen-binding fragment thereof.

The term "nucleic acid molecule" as used herein has a meaning comprehensively including DNA (gDNA and cDNA) and RNA molecules, and nucleotides, which are basic structural units in nucleic acid molecules, include natural nucleotides as well as analogues in which sugar or base sites are modified. The sequences of the nucleic acid molecules encoding the heavy and light chain variable regions of the present disclosure may be modified, wherein the modification includes additions, deletions, or non-conservative or conservative substitutions of nucleotides.

In addition, the present disclosure provides a recombinant expression vector including the nucleic acid molecule.

As used herein, the term "vector" refers to a self-replicating DNA molecule used to carry a clonal gene (or another piece of clonal DNA).

As used herein, the term "expression vector" refers to a recombinant DNA molecule including a desired coding sequence and an appropriate nucleic acid sequence essential for expressing a coding sequence operably linked in a specific host organism. The expression vector may preferably include one or more selectable markers. The marker is a nucleic acid sequence that may be selected by conventional chemical methods, and includes all genes capable of distinguishing transformed cells from non-transformed cells. Examples thereof include antibiotic resistant genes such as ampicillin, kanamycin, geneticin (G418), bleomycin, hygromycin, and chloramphenicol, but are limited thereto and appropriately selected by those skilled in the art.

To express the DNA sequences of the present disclosure, any of a wide variety of expression regulatory sequences may be used in the vector. Examples of useful expression regulatory sequences may include, for example, early and late promoters of SV40 or adenovirus, promoters and enhancers of CMV, LTR of retrovirus, lac system, trp system, TAC or TRC system, T3 and T7 promoters, major operator and promoter regions of phage lambda, regulatory regions of fd code proteins, promoters for 3-phosphoglycerate kinase or other glycolytic enzymes, promoters of the phosphatases such as Pho5, promotors in yeast alpha-crossing system and other sequences of construction and induction known to regulate the expression of genes in prokaryotic or eukaryotic cells or viruses thereof, and various combinations thereof.

The vector expressing the antibody of the present disclosure may be a vector system in which a light chain and a heavy chain are simultaneously expressed in a single vector or a system in which a light chain and a heavy chain are expressed in separate vectors. For the latter case, both vectors are transfected into the host cell through co-transformation and targeted transformation. Co-transformation is a method of screening cells expressing both the light and heavy chains after co-introducing each vector DNA encoding the light and heavy chains into a host cell. Targeted transformation is a method of screening cells transformed with a vector including a light chain (or heavy chain) and then re-transforming the screened cells expressing the light chain with a vector including a heavy chain (or light chain) so as to finally screen cells expressing both the light chain and the heavy chain.

In addition, the present disclosure provides a cell transformed with a recombinant expression vector.

Cells capable of stably and continuously cloning and expressing the vector of the present disclosure may be any host cells known in the art, including prokaryotic host cells such as Escherichia coli, Bacillus sp. strains such as Bacillus subtilis and Bacillus thuringiensis, Streptomyces, Pseudomonas (e.g., Pseudomonas putida), Proteus mirabilis or Staphylococcus (e.g., Staphylocus carnosus), but are not limited thereto.

In the method of preparing the antibody or antigen-binding fragment thereof, the transformed cells may be cultured depending on an appropriate medium and culture conditions known in the art. Such the culture process may be easily adjusted and used by those skilled in the art depending on the selected strain. Cell culture is divided into suspension culture and adherent culture according to the cell growth method, and also into batch, fed-batch and continuous culture methods according to the culture method. Media used for culture should suitably satisfy the requirements of a specific strain.

In addition, the present disclosure provides a composition for diagnosing cancer, including the antibody or antigen-binding fragment thereof as an active ingredient.

Preferably, the cancer may be a cancer in which LGALS3BP is expressed, and more preferably, the cancer may be pancreatic cancer, but is not limited thereto.

In addition, the present disclosure provides a pharmaceutical composition for preventing or treating cancer, including the antibody or antigen-binding fragment thereof as an active ingredient.

Preferably, the cancer may be a cancer in which LGALS3BP is expressed, and more preferably, the cancer may be a pancreatic cancer, but is not limited thereto.

In addition, the present disclosure provides a pharmaceutical composition for preventing or treating cancer metastasis, including the antibody or antigen-binding fragment thereof as an active ingredient.

Preferably, the cancer may be a pancreatic cancer, but is not limited thereto.

The pharmaceutical composition of the present disclosure may further include a pharmaceutically acceptable carrier, wherein the pharmaceutically acceptable carrier is commonly used in formulation, including lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methylcellulose, methylhydroxybenzoate, propylhydroxybenzoate, talc, magnesium stearate, and mineral oil, but is not limited thereto. The composition for preventing or treating cancer metastasis of the present disclosure may further include a lubricant, wetting agent, sweetener, flavoring agent, emulsifier, suspending agent, and preservative in addition to the above components.

The pharmaceutical composition of the present disclosure may be administered orally or parenterally, and in the case of parenteral administration, administration may be performed by intravenous injection, subcutaneous injection, intramuscular injection, intraperitoneal injection, endothelial administration, topical administration, intranasal administration, intrapulmonary administration, and rectal administration. When administered orally, since the protein or peptide is digested, oral compositions may be prepared by coating active agents or formulated to protect from degradation in the stomach, and the composition of the present disclosure may be administered by any device that allows the active ingredient to be delivered to a target cell.

A suitable dosage of the pharmaceutical composition of the present disclosure varies depending on factors such as formulation method, administration type, age, weight, sex, pathological condition, food, administration duration, administration route, excretion rate, and response sensitivity of a patient, and a skilled physician may easily determine and prescribe an effective dosage for the desired treatment or prevention.

The pharmaceutical composition of the present disclosure is prepared in a unit dosage form by formulating with a pharmaceutically acceptable carrier and/or excipient according to a method that may be easily carried out by those of ordinary skill in the art to which the present disclosure pertains, or it may be prepared by being put into a multi-dose container. In this case, the formulation may be in the form of a solution, suspension or emulsion in oil or aqueous medium or in the form of an extract, powder, suppository, powder, granule, tablet, or capsule, and may additionally include a dispersant or stabilizer.

The composition of the present disclosure may be administered as an individual therapeutic agent or in combination with other therapeutic agents and administered sequentially or simultaneously with conventional therapeutic agents.

### Modes for Carrying Out the Invention

Hereinafter, the present disclosure will be described in more detail through example embodiments. These example embodiments are only for illustrating the present disclosure in more detail, and it will be apparent to those skilled in the art that the scope of the present disclosure is not limited by these example embodiments according to the gist of the present disclosure.

### <Experimental example>

The following experimental examples are intended to provide experimental examples commonly applied to each embodiment according to the present disclosure.

### 1. Analysis of migration, invasion and proliferation

Migration and infiltration ability were analyzed respectively using Transwell chambers (Corning Costar) and matrigel-coated chambers with 6.5-mm diameter polycarbonate filters (8 µm pore size). Cells were trypsinized and suspended in serum-free culture medium at a final concentration of 1 × 10⁶ cells/mL. 100 µl of cell suspension was loaded to each top well, and the culture medium was added to the plate as a chemotactic. The chamber was subjected to a reaction at 37°C for 24 hours. Cells were immobilized and stained with hematoxylin and eosin. Non-migrating cells in the upper chamber were removed with a cotton swab. The number of migrated and infiltrated cells was quantified by counting the cells under a light microscope (×200).

Cell viability was measured using Ezcytox according to the manufacturer's protocol. Cells were inoculated by 2 × 10³ cells/100 µl/96 well, followed by culture at 37°C for 72 hours. The absorbance of the well was measured at 450 nm compared to the control wavelength of 650 nm using a Molecular Devices microplate reader.

### 2. In vivo tumor formation and image analysis

110621 cells (2 × 10⁶) re-suspended in 100 µL ofPBS were injected into the dorsal flank of 6- to 8-week-old male BALB/c nude mice. Tumor growth was observed and volume was measured using a caliper. Tumor size (mm³) = L (length) × W² (width)/2. Subcutaneous tumors were excised and weighed. Tumor tissues were fixed with formalin, embedded with paraffin, and stained with H&E. Immunohistochemical staining for detection of proliferating cells was performed using Ki-67 antibody, and positively stained cells were calculated by counting the number of immune-positive cells in 5 selective areas.

### 3. Immunohistochemical analysis

Fresh specimens were soaked in 10% formalin for 4 days and embedded in paraffin. 4-µm paraffin sections were de-paraffinized with xylene and rehydrated through step-by-step alcohol. Intrinsic peroxidase activity was blocked with 3% hydrogen peroxide dissolved in methanol for 20 minutes. Heat-mediated antigenic activation was performed with Tris-EDTA buffer (abcam) for 20 minutes. In order to block non-specific binding, after reacting with 3% normal goat serum for 20 minutes, the sections were reacted overnight with Ki67 antibody (abcam, 1:50) diluted in 1% normal goat serum. The sections were rinsed with PBS and reacted with HRP-conjugated secondary antibody (1:200) at room temperature for 1 hour. Finally, the sections were processed with diaminobenzidine (DAB, Thermo Scientific) and counterstained with hematoxylin. After mounting a coverslip using Permount (Fisher Scientific), observation was performed with a light microscope, followed by analysis using Image J program.

### 4. Phage display for Gal-3BP antibody screening

Three white leghorn chickens were immunized and boosted three times with recombinant Gal-3BP protein. After the third boost, chickens were sacrificed, and spleens, bursae of Fabricius and bone marrow were collected for total RNA isolation using TRIzol Reagent (Thermo Fisher Scientific). Using the SuperScript IV First-Strand Synthesis System (Thermo Fisher Scientific), complementary DNA was synthesized according to the manufacturer's protocol. Using cDNA as a template, genes encoding variable regions of the heavy and light chain were amplified to be used to construct a chicken scFv-display phage library. The chicken scFv-display phage library was subjected to 4 rounds of biopanning against Gal-3BP protein. Briefly, the scFv phage-display library was added to recombinant Gal-3BP recombinant protein conjugated with 3 µg of 5.0 × 10⁶ magnetic beads (Dynabeads M-270 epoxy, Invitrogen), followed by a reaction with involvement of rotation at 37°C for 2 hours. During biopanning, beads were washed with 500 µL of 0.05% (v/v) Tween-20 (Sigma-Aldrich, St. Louis, MO, USA)(PBST) dissolved in PBS. Phage bound to the beads were eluted, neutralized, infected to E. coli ER2738 (New England Biolabs, Ipswich, MA, USA) and rescued. scFv clones were randomly selected from the last round of output titer plates and subjected to phage enzyme immunoassay using Gal-3BP-coated microtiter plates (Corning Life Sciences, NY, USA). The nucleotide sequences of reactive scFv clones were determined by Sanger sequencing (Cosmogenetech, Seoul, Korea).

### <Example 1> Discovery of LGALS3BP using proteomic technique in cancer tissues of a pancreatic cancer patient-derived xenograft model.

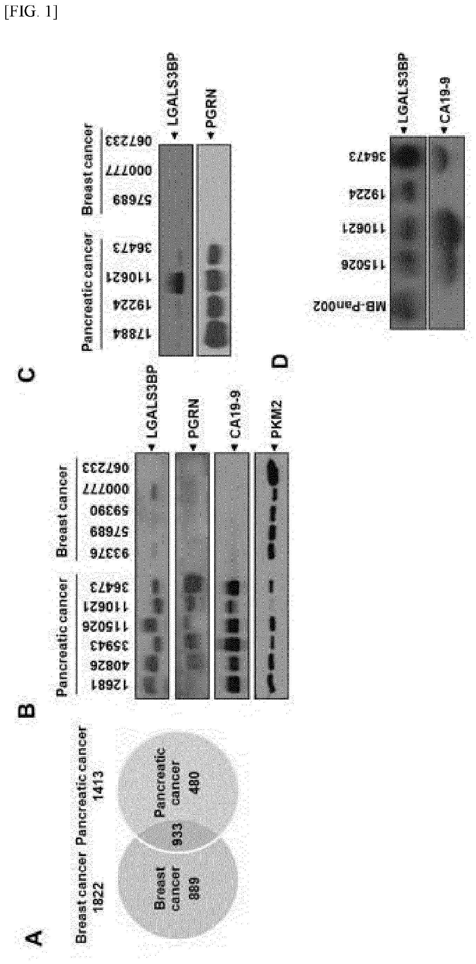

In pancreatic cancer and breast cancer, specific proteins for each cancer type were discovered using the proteomic technique, and the pancreatic cancer-specific expression of LGALS3BP was verified through document review. In addition, it was found that it is also secreted from the culture medium of cancer cells and expressed in the plasma of a mouse model transplanted with cancer (FIG. 1).

### <Example 2> Identification of inhibition of cancer development and growth upon inhibition of LGALS3BP expression in pancreatic cancer cells

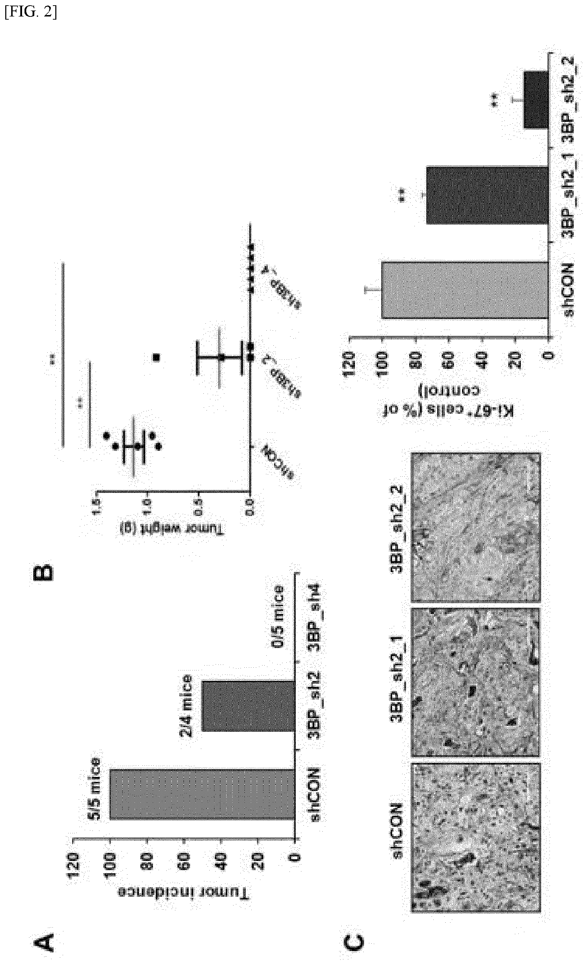

After inhibiting the expression of LGALS3BP in patient-derived pancreatic cancer cells and transplanting into mice, it was found that the cancer development was significantly reduced, the weight of the generated cancer was reduced, and the ki67 index expressing cancer cell proliferation was also reduced (FIG. 2).

### <Example 3> Identification of reduced cell migration and invasion ability upon inhibition of LGALS3BP expression in pancreatic cancer cells

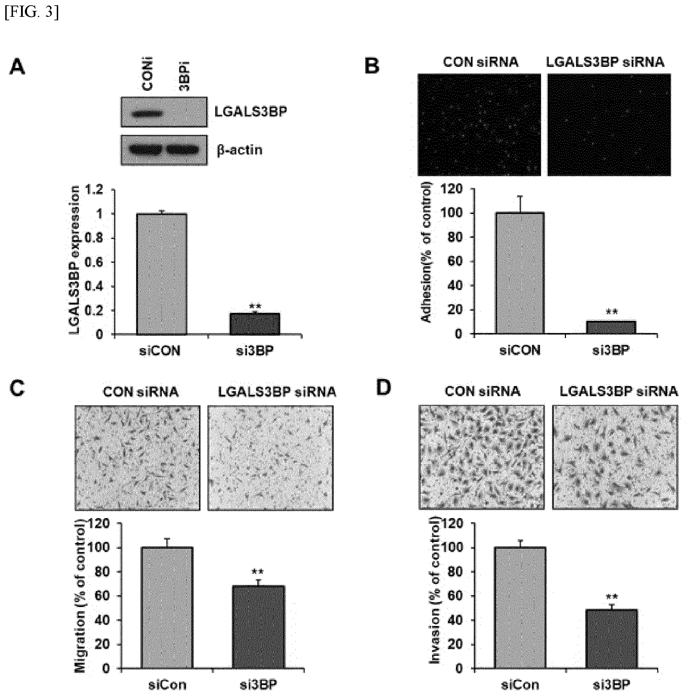

When LGALS3BP was stably inhibited, it was observed that the surface anchoring ability, migration ability, and invasion ability of the patient-derived pancreatic cancer cells were significantly reduced upon inhibition of LGALS3BP expression (FIG. 3).

### <Example 4> Discovery of LGALS3BP antibody using phage display technique

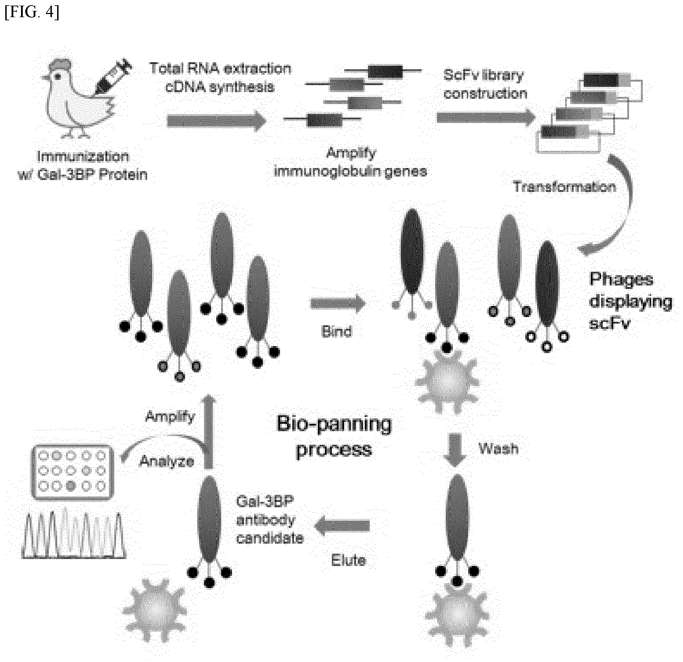

For the purpose of developing an anticancer drug that inhibits LGALS3BP, the task of discovering a specific binding antibody was carried out, and candidates with high binding ability were screened via the phage display method (FIG. 4).

Specifically, LGALS3BP was produced and purified in E. coli and then injected into chickens (immunization) to induce antibody production. RNA was extracted from the chicken, and cDNA of the antibody portion was synthesized and expressed in phagemid in the form of a single-chain antibody. This is called a phage library, and the library was bound to LGALS3BP to screen only antibodies having high binding ability. This is called bio-panning as an in vitro binding method similar to ELISA.

As a result, as shown in Table 1, 5 antibodies with high binding ability were screened as follows (a result of ELISA).

**TABLE 1**

| **CH2-18** | **CH2-19** | **CH2-44** | **CH2-67** | **CH2-84** |
|---|---|---|---|---|
| **1.0114** | **0,3682** | **04129** | **2,1939** | **1,1699** |
| **1.128** | **1.3764** | **1.142** | **2.609** | **0.5577** |
| | **1.3301** | **0.77** | **0.5791** | |
| | **1.4122** | | **2.0092** | |

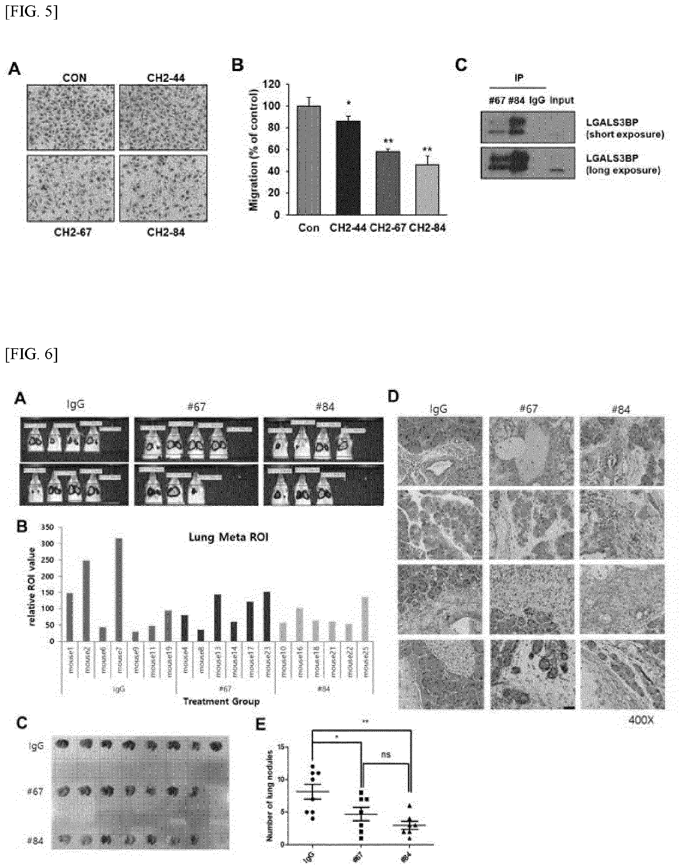

Through functional analysis of these antibody candidates, it was found that antibodies #67 and #84 had excellent binding ability and reduced the migration ability of cancer cells (FIG. 5).

The amino acid sequences and nucleotide sequences of antibodies #67 and #84 are shown in Tables 2 to 5.

**TABLE 2**

| CH2-67 | Amino acid sequence of LGALS3BP #67 antibody |
|---|---|
| CDRH1 | GFTFSSYGLG (SEQ ID NO: 1) |
| CDRH2 | GISSGGATFYGAAMKG (SEQ ID NO: 2) |
| CDRH3 | DARSGSWSPDAGQIDA (SEQ ID NO: 3) |
| CDRL1 | SGSSGSHYG (SEQ ID NO: 4) |
| CDRL2 | SNDKRPS (SEQ ID NO: 5) |
| CDRL3 | GSIDSSGI (SEQ ID NO: 6) |
| VH | AVTLDESGGGLQTPGGALSLVCKGSGFTFSSYGLGWVRQAPGKGLEY VAGISSGGATFYGAAMKGRATISRDNGQSTVRLQLNNLRAEDTATYY CAKDARSGSWSPDAGQIDAWGHGTEVIVSSTS (SEQ ID NO: 7) |
| VL | LTQPSSVSANLGGTVEITCSGSSGSHYGWFQQKSPGSAPVTLIYSNDKR PSDIPSRFSGSKSGSTATLTITGVQAEDEAVYFCGSIDSSGIFGAGTTLTV L (SEQ ID NO: 8) |

**TABLE 3**

| CH2-67 | Nucleotide sequence of LGALS3BP #67 antibody |
|---|---|
| VH | GCC GTG ACG TTG GAC GAG TCC GGG GGC GGC CTC CAG ACG CCC GGA GGA GCG CTC AGC CTC GTC TGC AAG GGC TCC GGG TTC ACC TTC AGC AGT TAT GGC CTG GGT TGG GTG CGA CAG GCG CCC GGC AAG GGG CTC GAG TAC GTT GCG GGT ATT AGT AGC GGT GGT GCA ACA TTC TAC GGG GCG GCG ATG AAG GGC CGT GCC ACC ATC TCG |
| | AGG GAC AAC GGG CAG AGC ACA GTG AGG CTG CAG CTG AAC AAC CTC AGG GCT GAG GAC ACC GCC ACC TAC TAC TGC GCC AAA GAT GCT CGC AGT GGT AGT TGG AGT CCT GAT GCT GGT CAA ATC GAC GCA TGG GGC CAC GGG ACC GAA GTC ATC GTC TCC TCC ACT AGT (SEQ ID NO:17) |
| VL | CTG ACT CAG CCG TCC TCG GTG TCA GCA AAC CTG GGA GGA ACC GTC GAG ATC ACC TGC TCC GGG AGT AGT GGC AGC CAC TAT GGC TGG TTC CAG CAG AAG TCA CCT GGC AGT GCC CCT GTC ACT CTG ATC TAT AGC AAC GAC AAG AGA CCC TCG GAC ATC CCT TCA CGA TTC TCC GGT TCC AAA TCC GGC TCC ACA GCC ACA TTA ACC ATC ACT GGG GTC CAA GCC GAG GAC GAG GCT GTC TAT TTC TGT GGG AGC ATA GAC AGC AGT GGT ATA TTT GGG GCC GGG ACA ACC CTG ACC GTC CTA (SEQ ID NO: 18) |

**TABLE 4**

| CH2- 84 | Amino acid sequence of LGALS3BP #84 antibody |
|---|---|
| CDRH1 | GFTFNGYGMN (SEQ ID NO: 9) |
| CDRH2 | GIDDTGSYTAYAPAVRG (SEQ ID NO: 10) |
| CDRH3 | GYGDVWGGDEIDA (SEQ ID NO: 11) |
| CDRL1 | SGGSSGYG (SEQ ID NO: 12) |
| CDRL2 | DNDKRPS (SEQ ID NO: 13) |
| CDRL3 | GGYDNSVGI (SEQ ID NO: 14) |
| VH | AVTLDESGGGLQTPGGALSLVCKASGFTFNGYGMNWVRQAPGKGLE WVAGIDDTGSYTAYAPAVRGRATISRDNGQSTVRLQLNNLRAEDTAT YYCAKGYGDVWGGDEIDAWGHGTEVIVSSTS (SEQ ID NO: 15) |
| VL | LTQPSSVSANLGGTVKITCSGGSSGYGWHQQKSPGSAPVTVIYDNDKR PSDIPSRFSGSLSGSTNTLTITGVQAEDEAVYFCGGYDNSVGIFGAGTTL TVL (SEQ ID NO: 16) |

**TABLE 5**

| CH2-84 | Nucleotide sequence of LGALS3BP #84 Antibody |
|---|---|
| VH | GTG ACG TTG GAC GAG TCC GGG GGC GGC CTC CAG ACG CCC GGA GGA GCG CTC AGC CTC GTC TGC AAG GCC TCC GGG TTC ACC TTC AAC GGT TAC GGC ATG AAC TGG GTG CGA CAG GCG CCC GGC AAG GGG CTG GAG TGG GTC GCT GGT ATT GAT GAC ACT GGT AGT TAC ACA GCA TAC GCG CCG GCG GTG AGG GGC CGT GCC ACC ATC TCG AGG GAC AAC GGG CAG AGC ACA GTG AGG CTG CAG CTG AAC AAC CTC AGG GCT GAG GAC ACC GCC ACC TAC TAC TGC GCC AAA GGT TAT GGT GAT GTT TGG GGT GGT GAT GAG ATC GAC GCA TGG GGC CAC GGG ACC GAA GTC ATC GTC TCC TCC ACT AGT (SEQ ID NO: 19) |
| VL | CTG ACT CAG CCG TCC TCG GTG TCA GCA AAC CTG GGA GGA ACC GTC AAG ATC ACC TGC TCC GGG GGT AGC AGC GGC TAT GGC TGG CAC CAG CAG AAA TCA CCT GGC AGT GCC CCT GTC ACT GTG ATC TAT GAC AAC GAC AAG AGA CCC TCG GAC ATC CCT TCA CGA TTC TCC GGT TCC CTA TCC GGC TCC ACA AAC ACA TTA ACC ATC ACT GGG GTC CAA GCC GAG GAC GAG GCT GTC TAT TTC TGT GGT GGC |
| | TAC GAC AAC AGT GTC GGT ATA TTT GGG GCC GGG ACA ACC CTG ACC GTC CTA (SEQ ID NO: 20) |

### <Example 5> Verification of inhibitory effect of the LGALS3BP antibody on cancer growth and metastasis in an animal model with pancreatic cancer

In a pancreatic orthotopic transplant model using patient-derived pancreatic cancer cells, the anticancer effects of the two antibodies were tested via IVIS. As shown in FIG. 6, it was found that the size of cancer was reduced in the group to which the antibody was administered, and by administering the antibody in the lung metastasis model, a significant decrease was observed in the proportion of cancer cells or lung nodules (FIG. 6).

### <Example 6> Sequencing for humanization of LGALS3BP antibody

Sequencing for humanization of anti-LGalS3BP antibody CH2-84 was performed.

Human germline genes most similar to CH2-84; Sequencing for humanization was performed using the following human genes. The sequencing was carried out only for the FRs, not the CDRs.

**TABLE 6**

| Gene segment | Human genes and alleles | No. of different residues; Ch vs Hu |
|---|---|---|
| Light chain V gene | IGLV3-25*02 | 18 |
| Light chain J gene | IGLJ2* 01 | 2 |
| Heavy chain V gene | IGHV3-NL1 * 01 | 21 |
| Heavy chain J gene | IGHJ 1 * 01 | 3 |

As a result, a total of three candidate clones were secured.
1) 2 chicken sequences left with Hu2-84_11-7; VL11 & VH7 combination.
2) 3 chicken sequences left with Hu2-84_12-7; VL12 & VH7 combination.
3) 3 chicken sequences left with Hu2-84_13-7; VL13 & VH7 combination.

The VL, VH and closest human germline sequences of each of the human sequenced antibodies are as follows. CDRs are underlined and non-human sequenced chicken-derived residues are bolded.
>IGHV3-NL1^{∗}01_IGHJ1^{∗}01 01

QVQLVESGGG VVQPGGSLRL SCAAS_____ ___MHWVRQA
PGKGLEWVS_ __________ ______RFTI SRDNSKNTLY LQMNSLRAED
TAVYYCAK__ __________ _WGQGTLVTV SS

>VH7

QVQLVESGGG VVQPGGSLRL SCAASGFTFN GYGMNWVRQA
PGKGLEWVSG IDDTGSYTAY APAVRGRFTI SRDNSKNTLY LQMNSLRAED
TAVYYCAKGY GDVWGGDEID AWGQGTLVTV SS (SEQ ID NO: 21)

>IGLV3-25*02_IGLJ2*01 
ELTQPPSVSV SPGQTARITC ________WY QQKPGQAPVL VIY_______
GIPERFSGSS SGTTVTLTIS GVQAEDEADY YC________ _FGGGTKLTV L
>VL11 
ELTQPPSVSV SPGQTARITC SGGSSGYGWH QQKPGQAPVL
VIYDNDKRPS GIPERFSGSS SGTTVTLTIS GVQAEDEADY YCGGYDNSVG
IFGGGTKLTV L (SEQ ID NO: 22)
>VL12 
ELTQPPSVSV SPGQTARITC SGGSSGYGWH QQKPGQAPVL
VIYDNDKRPS GIPERFSGSL SGTTVTLTIS GVQAEDEADY YCGGYDNSVG
IFGGGTKLTV L (SEQ ID NO: 23)
>VL13

ELTQPPSVSV SPGQTARITC SGGSSGYGWH QQKPGQAPVL
VIYDNDKRPS GIPERFSGSS SGSTVTLTIS GVQAEDEADY YCGGYDNSVG
IFGGGTKLTV L (SEQ ID NO: 24)

### <Example 7> Verification of metastatic ability inhibitory effect of humanized LGALS3BP antibody

In order to verify whether the humanized antibody has the same metastatic inhibitory effect as the conventional CH2-84, a migration assay was performed with cells derived from pancreatic cancer patients.

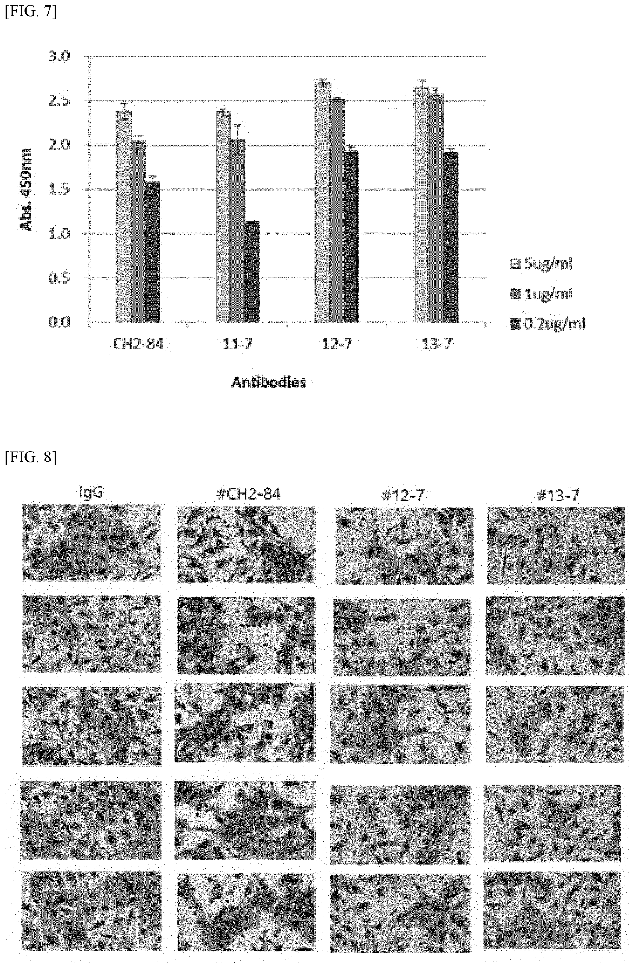

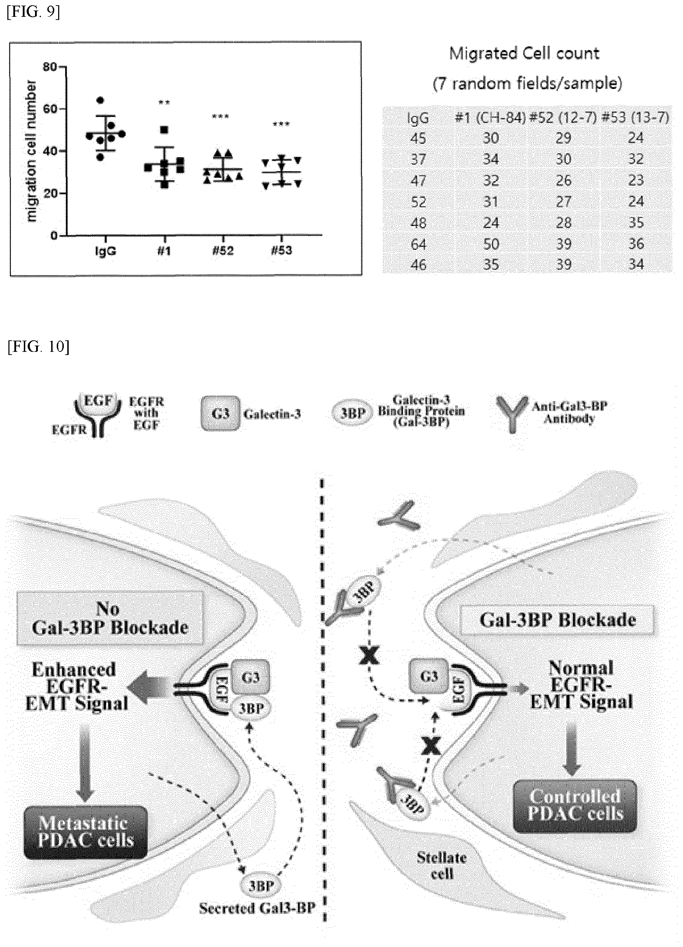

As a result, as shown in FIGS. 8 and 9, it was found that the two humanized antibodies had the ability to inhibit cancer cell metastasis in a degree similar to that of conventional CH2-84.

As described above, a specific part of the content of the present disclosure is described in detail, for those of ordinary skill in the art, it is clear that the specific description is only a preferred embodiment, and the scope of the present disclosure is not limited thereby. Accordingly, the substantial scope of the present disclosure may be defined by the appended claims and their equivalents.

## Claims

1. An antibody or antigen-binding fragment thereof specifically binding to galectin 3-binding protein (LGALS3BP), comprising a heavy chain variable region comprising a heavy chain CDR1 having an amino acid sequence represented by SEQ ID NO: 1, a heavy chain CDR2 having an amino acid sequence represented by SEQ ID NO: 2, and a heavy chain CDR3 having an amino acid sequence represented by SEQ ID NO: 3; and a light chain variable region comprising a light chain CDR1 having an amino acid sequence represented by SEQ ID NO: 4, a light chain CDR2 having an amino acid sequence represented by SEQ ID NO: 5, and a light chain CDR3 having an amino acid sequence represented by SEQ ID NO: 6.

2. The antibody or antigen-binding fragment thereof of claim 1, wherein the heavy chain variable region comprises an amino acid sequence represented by SEQ ID NO: 7, and the light chain variable region comprises an amino acid sequence represented by SEQ ID NO: 8.

3. An antibody or antigen-binding fragment thereof specifically binding to LGALS3BP, comprising a heavy chain variable region comprising a heavy chain CDR1 having an amino acid sequence represented by SEQ ID NO: 9, a heavy chain CDR2 having an amino acid sequence represented by SEQ ID NO: 10, and a heavy chain CDR3 having an amino acid sequence represented by SEQ ID NO: 11; and a light chain variable region comprising a light chain CDR1 having an amino acid sequence represented by SEQ ID NO: 12, a light chain CDR2 having an amino acid sequence represented by SEQ ID NO: 13, and a light chain CDR3 having an amino acid sequence represented by SEQ ID NO: 14.

4. The antibody or antigen-binding fragment thereof of claim 3, wherein the heavy chain variable region comprises an amino acid sequence represented by SEQ ID NO: 15, and the light chain variable region comprises an amino acid sequence represented by SEQ ID NO: 16.

5. The antibody or antigen-binding fragment thereof of claim 3, wherein the heavy chain variable region comprises an amino acid sequence represented by SEQ ID NO: 21, and the light chain variable region comprises an amino acid sequence represented by SEQ ID NO: 22, an amino acid sequence represented by SEQ ID NO: 23, or an amino acid sequence represented by SEQ ID NO: 24.

6. The antibody or antigen-binding fragment thereof of claim 5, wherein the antibody is a humanized antibody.

7. A nucleic acid molecule encoding the antibody or antigen-binding fragment thereof of any one of claims 1 to 6.

8. A recombinant expression vector comprising the nucleic acid molecule of claim 7.

9. A cell transformed with the recombinant expression vector of claim 8.

10. A composition for diagnosing cancer, comprising the antibody or antigen-binding fragment thereof of any one of claims 1 to 6 as an active ingredient.

11. The composition of claim 10, wherein the cancer is a cancer in which LGALS3BP is expressed.

12. The composition of claim 11, wherein the cancer is a pancreatic cancer.

13. A pharmaceutical composition for preventing or treating cancer, comprising the antibody or antigen-binding fragment thereof of any one of claims 1 to 6 as an active ingredient.

14. The pharmaceutical composition of claim 13, wherein the cancer is a cancer in which LGALS3BP is expressed.

15. The pharmaceutical composition of claim 14, wherein the cancer is a pancreatic cancer.

16. A pharmaceutical composition for preventing or treating cancer metastasis, comprising the antibody or antigen-binding fragment thereof of any one of claims 1 to 6 as an active ingredient.

17. The pharmaceutical composition of claim 16, wherein the cancer is a pancreatic cancer.
